# EUROPÄISCHE PATENTSCHRIFT

(11) **EP 0 910 379 B1**
(45) Veröffentlichungstag und Bekanntmachung des Hinweises auf die Patenterteilung: **23.04.2003**
(21) Anmeldenummer: 97933646.8
(22) Anmeldetag: 02.07.1997
(51) Int. Cl.: A61K 31/48, A61K 9/70

(54) **PFLASTER ZUR TRANSDERMALEN ANWENDUNG VON PERGOLID**
PLASTER FOR THE TRANSDERMAL APPLICATION OF PERGOLIDE
EMPLATRE POUR L'UTILISATION TRANSDERMIQUE DE PERGOLIDE

(30) Priorität: 02.07.1996 DE 19626621
(43) Veröffentlichungstag der Anmeldung: 28.04.1999
(73) Patentinhaber: HEXAL AG, D-83607 Holzkirchen (DE)
(72) Erfinder: FISCHER, Wilfried, D-83607 Holzkirchen (DE); SENDL-LANG, Anna, D-83607 Holzkirchen (DE); ZEH-HERWERTH, Dagmar, D-83607 Holzkirchen (DE)
(74) Vertreter: Boeters, Hans Dietrich, Dr.
(86) Internationale Anmeldenummer: EP9703458
(87) Internationale Veröffentlichungsnummer: WO98000142

(56) Entgegenhaltungen:
- EP-A- 0 003 667
- EP-A- 0 204 954
- EP-A- 0 458 640
- WO-A-89/09599
- WO-A-91/00746
- WO-A-91/16885
- WO-A-96/04910
- WO-A-96/40139
- DE-A- 4 240 798
- GB-A- 2 204 240
- US-A- 4 797 405
- US-A- 5 114 948

## Beschreibung

Pergolid (D-6-n-Propyl-8β-methylmercaptomethylergolin) ist. ein Dopaminrezeptor-Agonist und wird unter anderem als Anti-Parkinsonmittel (EP-A-0 003 667), zur Behandlung von Sucht, die durch Psychostimulantien hervorgerufen wird (EP-A-0 204 954), und bei Nikotinabhängigkeit eingesetzt (GB-A-2 204 240).

Aus EP-A-0 003 667 und EP-A-0 527 835 sind verschiedene oral zu verabreichende Formulierungen von Pergolid bekannt. Die therapeutich wirksame Tagesdosis liegt bei 0,01 bis 20 mg.

Allgemein läßt sich sagen, daß die Bioverfügbarkeit von oral oder intravenös applizierten Wirkstoffen jedoch oft unbefriedigend ist. Die hepatische Metabolisierung des Wirkstoffs bei der ersten Leberpassage kann zu unerwünschten Konzentrationsverhältnissen und toxischen Nebenprodukten führen, zu Wirkverlust oder Verminderng der Wirkung. Gegenüber oraler Verabreichung besitzt die transdermale Gabe von Wirkstoffen verschiedene Vorteile. Die Wirkstoffzufuhr läßt sich über einen längeren Zeitraum besser steuern, wodurch hohe Blutplasmaschwankungen vermieden werden. Zudem kann die erforderliche therapeutisch wirksame Dosis meist deutlich verringert werden. Außerdem wird ein Pflaster vom Patienten oft mehr bevorzugt als täglich ein- oder mehrfach einzunehmende Tabletten.

Pergolid hat bei oraler Verabreichung eine geringe Bioverfügbarkeit. Demzufoge ist es schwierig, über einen längeren Zeitraum hinaus konstante Blutplasmaspiegel zu erreichen, so daß drei Tagesdosen erforderlich sind.

EP 0 458 640 schlägt beispielsweise Pergolid für eine transdermale Application vor, wobei jedoch die orale Route bevorzugt wird. Permeationsförderer für eine transdermale Applikation werden nicht genannt. Diesem Stand der Technik ist jedoch bereits zu entnehmen, dass es für eine erhöhte Permeation auf den Einsatz eines Permeationsförderers ankommt. Bei der Wahl eines geeigneten Permeationsförderers für Pergolid wäre der Fachmann jedoch vor eine praktisch unüberschaubare Zahl von Möglichkeiten gestellt. Analog schlägt DE 4 240 798 Pergolid zur percutanen Verabreichung vor, ohne widerum Permeationsförderer zu benennen.

WO 96/40 139 (Dokument gemäß Artikel 54 (3) EPÜ) beschreibt ein transdermales therapeutisches System mit Pergolid als Wirkstoff und verschiedenen Permeationsförderern.

Aufgabe der vorliegenden Erfindung ist es nun, ein transdermales System für die systemische Zufuhr von Pergolid oder einem seiner pharmazeutisch unbedenklichen Salze bereit zu stellen, mit dem die Schwächen oraler Verabreichungsformen vermieden werden sollen und für transdermale Systeme eine gegenüber dem Stand der Technik erhöhte Permeation erreicht wird.

Die der Erfindung zugrundeliegende Aufgabe wird nun durch ein transdermales therapeutisches System mit einem Gehalt an Pergolid oder einem seiner pharmazeutisch unbedenklichen Salze und an natürlichem Vitamin E, synthetischem Vitamin E und/oder einem Vitamin E-Derivat als Permeationsförderer gelöst.

Der Wirkstoff Pergolid kann dabei als freie Pergolid-Base, Pergolid-Mesylat und/oder Pergolid-Hydrochlorid zur Anwendung kommen.

Pergolid oder eines seiner pharmazeutisch verträglichen Salze als Wirkstoff kann ferner in Kombination mit einem oder mehreren anderen Dopamin-Agonisten angewendet werden, insbesondere in zwei- oder dreifacher Kombination. Dieses weiteren bekannten Wirkstoffe können die Pergolid-Wirkung modifizieren, verstärken, synergisieren oder potenzieren.

Bei dem weiteren Wirkstoff kann es sich insbesondere um Levodopa, Carbidopa, Selegelin, Tacrin, Physostigmin, Galanthamin, 1-Hydroxytacrin und/oder chemische Derivate derselben, Metabolite derselben und/oder parmazeutisch verträgliche Salze derselben handeln.

Als weiterer Permeationsförderer lassen sich einund/oder mehrwertige aliphatische, cycloaliphatische und/oder aromatisch-aliphatische Alkohole mit jeweils bis zu 8 C-Atomen, beispielsweise Ethanol, 1,2-Propandiol, Dexpantheonol und/oder Polyethylenglykol; Alkohol/Wasser-Gemische; gesättigte und/oder ungesättigte Fettalkohole mit jeweils 8 bis 18 Kohlenstoffatomen; gesättigte und/oder ungesättigte Fettsäuren mit jeweils 8 bis 18 Kohlenstoffatomen; und/oder deren Ester verwenden.

Da Pergolid zu einem gewissen Maß lichtempfindlich ist, können Stabilisatoren verwendet werden, wie sie beispielsweise aus US-A-5 114 948 oder EP-B-O 314 387 bekannt sind, beispielsweise Polyvinylpyrrolidon, alpha-Tocopherolsuccinat, Propylgallat, Methionin, Cystein und/oder Cystein-hydrochlorid.

Bei dem erfindungsgemäßen transdermalen therapeutischen System kann es sich um ein Pflaster handeln, vor allem mit undurchlässiger Deckschicht und abziehbarer Schutzschicht, insbesondere ein Matrixsystem oder ein Membransystem.

Für die Deckschicht kommen Polyester, Polypropylen, Polyethylen oder Polyurethan in Betracht, gegebenenfalls jeweils metallisiert oder pigmentiert, und für die abziehbare Schutzschicht Polyester, Polypropylen oder Papier mit Silikon- und/oder Polyethylenbeschichtung.

Bei dem erfindungsgemäßen transdermalen therapeutischen System kann es sich um ein Matrixpflaster mit
- einer undurchlässigen Deckschicht,
- einer wirkstoffhaltigen selbstklebenden Matrixschicht oder einer wirkstoffhaltigen Matrixschicht, die mit einem Haftkleber beschichtet ist,
- einer abziehbaren Schutzschicht,
- Pergolid oder einem seiner pharmazeutisch verträglichen Salze als Wirkstoff und
- natürlichem Vitamin E, synthetischem Vitamin E und/oder einem Vitamin E-Derivat als Permeationsförderer handeln,
- gegebenenfalls neben weiteren Dopamin-agonisten und/oder neben weiteren Permeationsförderern und/oder Stabilisatoren.

Dabei kann eine Matrix auf Basis von Polyacrylat, Silikon, Polyisobutylen, Butylkautschuk, Styrol/Butadien-

Copolymerisat oder Styrol/Isopren-Copolymerisat vorgesehen werden. Derartige medizinisch übliche Matrixbildner sind im Copolymerisat oder Styrol/Isopren-Copolymerisat vorgesehen werden. Derartige medizinisch übliche Matrixbildner sind im Stand der Technik vorgegeben. Beispiele für Acrylatkleber sind DuroTak-Kleber.

Eine weitere erfindungsgemäße Ausführungsform betrifft ein Membransystem mit
- einer undurchlässigen Deckschicht,
- einem wirkstoffhaltigen Reservoir oder einer wirkstoffhaltigen Reservoirschicht,
- einer mikroporösen oder semipermeablen Membran,
- einer fakultativen Haftklebeschicht,
- einer abziehbaren Schutzschicht,
- Pergolid oder einem seiner pharmazeutisch verträglichen Salze,
- natürlichem Vitamin E, synthetischem Vitamin E und/oder einem Vitamin E-Derivat als Permeationsförderer und
- gegebenenfalls neben weiteren Dopamin-Agonisten und/oder neben weiteren Permeationsförderern und/oder Stabilisatoren, Emulgatoren, Verdickungsmitteln und/oder üblichen Membransystem- bzw. Reservoirpflaster-Hilfsmitteln.

Die wirkstoffhaltige Reservoirschicht kann also durch einen Zwischenraum vorgesehen werden, der zwischen Abdeckschicht und Membran ausgebildet wird. Das Reservoir ist mit Wirkstoff und fakultativen Hilfsstoffen gefüllt.

Für die Membran eignen sich inerte Polymere, insbesondere auf Basis von Polypropylen, Polyvinylacetat oder Silikon.

Sofern eine Membran vorgesehen ist, kann sie je nach Porengröße eine die Wirkstofffreisetzung kontrollierende Wirkung haben.

Nachstehend wird die Erfindung durch Beispiele näher erläutert.

### Beispiel 1 (Matrix-Pflaster)

Die folgenden Komponenten werden in einer ausreichenden Menge Ethylacetat dispergiert:

| | |
|---|---|
| Pergolid | 10 g |
| natürliches Vitamin E | 10 g |
| Propylenglycol | 15 g |
| Acrylatkleber (als 35-proz. Lösung in Ethylacetat), z. B. DuroTak 326-1753 | 65 g |

Die erhaltene Dispersion wird auf einer handelsüblichen Beschichtungsmaschine derart auf eine silikonisierte Polypropylenfolie aufgetragen, daß ein Flächengewicht der trockenen wirkstoffhaltigen Klebstoffmatrix von 100 g/m² resultiert. Danach wird auf einer Kaschierstation eine 50 µm starke Polyurethanfolie zukaschiert. Danach stanzt man aus dem Laminat 20 cm² oder gegebenenfalls 10, 30, 40 oder 50 cm² große Pflaster.

### Beispiel 2 (Reservoir-TTS)

Es werden die folgenden Komponenten in Ethanol/Wasser dispergiert:

| | |
|---|---|
| Pergolid (oder entsprechende Menge Pergolid-Salz, z. B. Pergolid-roesylat) | 5 bis 10 % |
| ggf. natürliches Vitamin E und/oder | |
| Polyvinylpyrrolidon als Stabilisator | |

Für das herzustellende Pflaster werden folgende Elemente vorgesehen:
Abdeckschicht aus z. B. Polyethylen
semipermeable Membran, z. B. CoTan 9711
Haftkleber für Haftklebeschicht, z. B. Duro-Tak 326-1753
abziehbare Schutzschicht, z. B. Gelroflex

In einem ersten Schritt wird mit Hilfe des Haftklebers, der Membran und der Schutzschicht auf einer üblichen Maschine ein Laminat hergestellt. In einem zweiten Schritt wird aus Laminat und Abdeckschicht ein Leer-TTS hergestellt. In einem dritten Schritt wird das Leer-TTS mit der Wirkstoffdispersion befüllt. In einem vierten Schritt wird das befüllte TTS verschlossen und in einem fünften Schritt werden Pflaster gewünchter Größe ausgestanzt.

## Patentansprüche

1. Transdermales therapeutisches System mit einem Gehalt an Pergolid oder einem seiner pharmazeutisch unbedenklichen Salze und an natürlichem Vitamin E, synthetischem Vitamin E und/oder einem Vitamin E-Derivat als Permeationsförderer.

2. Transdermales therapeutisches System nach Anspruch 1, **gekennzeichnet durch** die freie Pergolid-Base, Pergolid-mesylat und/oder Pergolid-hydrochlorid als Wirkstoff.

3. Transdermales therapeutisches System nach Anspruch 1 oder 2, **gekennzeichnet durch** mindestens einen weiteren üblichen Wirkstoff, nämlich mindestens einen anderen Dopamin-Agonisten.

4. Transdermales therapeutisches System nach Anspruch 3, **gekennzeichnet durch** Pergolid oder eins seiner pharmazeutisch verträglichen Salze als Wirkstoff in zwei- oder dreifacher Kombination mit anderen Dopamin-Agonisten.

5. Transdermales therapeutisches System nach einem der Ansprüche 3 oder 4, **gekennzeichnet durch** Levodopa, Carbidopa, Selegelin, Tacrin, Physostigmin, Galanthamin, 1-Hydroxytacrin und/oder chemische Derivate derselben, Metabolite derselben und/oder pharmazeutisch verträgliche Salze derselben.

6. Transdermales therapeutisches System nach einem der vorhergehenden Ansprüche, **gekennzeichnet durch** einen aliphatischen, cycloaliphatischen und/oder aromatischaliphatischen Alkohol, jeweils ein-oder mehrwertig und jeweils mit bis zu 8 C-Atomen, ein Alkohol/Wasser Gemisch, einen gesättigten und/oder ungesättigten Fettalkohol mit jeweils 8-18 C-Atomen, eine gesättigte und/oder ungesättigte Fettsäure mit jeweils 8-18 Kohlenstoffatomen und/oder deren Ester als weiteren Permeationsförderer.

7. Transdermales therapeutisches System nach Anspruch 6, **gekennzeichnet durch** Ethanol, 1,2-Propandiol, Dexpanthenol und/oder Polyethylenglykol als Permeationsförderer.

8. Transdermales therapeutisches System nach einem der vorhergehenden Ansprüche, **gekennzeichnet durch** einen bekannten Stabilisator, insbesondere Polyvinylpyrrolidon, alpha-Tocopherolsuccinat, Propylgallat, Methionin, Cystein und/oder Cystein-hydrochlorid.

9. Transdermales therapeutisches System nach einem der vorhergehenden Ansprüche in Form eines Pflasters mit einer undurchlässigen Deckschicht und einer abziehbaren Schutzschicht.

10. Transdermales therapeutisches System nach Anspruch 9, **dadurch gekennzeichnet, daß** das Pflaster ein Matrixsystem oder ein Membransystem ist.

11. Transdermales therapeutisches System nach Anspruch 9 oder 10, **gekennzeichnet durch** eine Deckschicht auf Basis von Polyester, Polypropylen, Polyethylen oder Polyurethan, gegebenenfalls jeweils metallisiert oder pigmentiert.

12. Transdermales therapeutisches System nach Anspruch 9, 10 oder 11, **gekennzeichnet durch** eine abziehbare Schutzschicht auf Basis von Polyester, Polypropylen oder Papier mit Silikonund/oder Polyethylenbeschichtung.

13. Transdermales therapeutisches System nach Anspruch 9, 10, 11 oder 12, **dadurch gekennzeichnet, daß** es sich um ein Matrixsystem mit
- einer undurchlässigen Deckschicht,
- einer wirkstoffhaltigen selbstklebenden Matrixschicht oder einer wirkstoffhaltigen Matrixschicht, die mit einem Haftkleber beschichtet ist,
- einer abziehbaren Schutzschicht und
- Pergolid oder einem seiner pharmazeutisch verträglichen Salze als Wirkstoff handelt.

14. Transdermales therapeutisches System nach Anspruch 13, **gekennzeichnet durch** eine Matrixschicht auf Basis von Polyacrylat, Silikon, Polyisobutylen, Butylkautschuk, Styrol/Butadien-Copolymerisat oder Styrol/Isopren-Copolymerisat.

15. Transdermales therapeutisches System nach Anspruch 9, 10, 11 oder 12, **dadurch gekennzeichnet, daß** es sich um ein Membransystem mit
- einer undurchlässigen Deckschicht,
- einem wirkstoffhaltigen Reservoir oder einer wirkstoffhaltigen Reservoirschicht,
- einer mikroporösen oder semipermeablen Membran,
- einer fakultativen Haftklebeschicht
- einer abziehbaren Schutzschicht und
- Pergolid oder einem seiner pharmazeutisch verträglichen Salze als Wirkstoff handelt.

16. Transdermales therapeutisches System nach Anspruch 15, **gekennzeichnet durch** bekannte Stabilisatoren, Emulgatoren, Verdickungsmittel und/oder bekannte Membransystem-Hilfsmittel.

17. Transdermales therapeutisches System nach Anspruch 15 oder 16, **gekennzeichnet durch** eine Membran auf Basis eines inerten Polymeren, insbesondere Polypropylen, Polyvinylacetat oder Silikon.

## Claims

1. Transdermal therapeutic system comprising an amount of pergolid or one of its pharmaceutically acceptable salts and of natural vitamin E, synthetic vitamin E and/or a vitamin E derivative as permeation promoter.

2. Transdermal therapeutic system of Claim 1, **characterized by** the free pergolid base, pergolid mesylate and/or pergolid hydrochloride as the active agent.

3. Transdermal therapeutic system of Claim 1 or 2, **characterized by** at least one further common active agent, viz. at least one other dopamine agonist.

4. Transdermal therapeutic system of Claim 3, **characterized by** pergolid or one of its pharmaceutically acceptable salts as active agent in two-fold or three-fold combination with another dopamine agonist.

5. Transdermal therapeutic system according to any of Claims 3 or 4, **characterized by** levodopa, carbidopa, selegeline, tacrine, physostigmine, galanthamine, 1-hydroxytacrine and/or chemcial derivatives thereof, metabolites thereof and/or pharmaceutically acceptable salts thereof.

6. Transdermal therapeutic system according to any one of the preceding claims, **characterized by** an aliphatic, cycloaliphatic and/or aromatic-aliphatic alcohol, which is single or multi-valent each and has up to 8 C atoms each, an alcohol-water mixture, a saturated and/or unsaturated fatty alcohol with 8 to 18 C atoms each, a saturated and/or unsaturated fatty acid with 8 to 18 C atoms each and/or its esters as further permeation promoter.

7. Transdermal therapeutic system according to Claim 6, **characterized by** ethanol, 1,2-propandiol, dexpanthenol and/or polyethylene glycol as the permeation promoter.

8. Transdermal therapeutic system of any one of the preceding claims, **characterized by** a known stabiliser, in particular polyvinylpyrrolidone, alpha-tocopherolsuccinate, propylgallate, methionine, cysteine and/or cysteine hydrochloride.

9. Transdermal therapeutic system of any one of the preceding claims in the form of a patch with an impermeable cover layer and a removeable protection layer.

10. Transdermal therapeutic system of Claim 9, **characterized in that** the patch is a matrix system or a membrane system.

11. Transdermal therapeutic system of claim 9 or 10, **characterized by** a cover layer on the basis of polyester, polypropylene, polyethylene or polyurethane, each optionally metallized or pigmented.

12. Transdermal therapeutic system of Claim 9, 10 or 11, **characterised by** a removeable protection layer on the basis of polyester, polypropylene or paper with a silicone and/or polyethylene coating.

13. Transdermal therapeutic system of Claim 9, 10, 11 or 12, characterized that the system is a matrix system including:
- an impermeable cover layer,
- a drug-containing self-adhesive matrix layer or a drug-containing matrix layer coated with an adhesive,
- removeable protection layer, and
- pergolid or one of its pharmaceutically acceptable salts as active agent.

14. Transdermal therapeutic system of Claim 13, **characterized by** a matrix layer on the basis of polyacrylate, silicone, polyisobutylene, butyl rubber, styrene/butadiene copolymer or styrene/isoprene copolymer.

15. Transdermal therapeutic system of Claim 9, 10, 11 or 12, characterized that the system is a membrane system including:
- an impermeable cover layer,
- an agent-containing reservoir or a drug-containing reservoir layer,
- a microporous or semi-permeable membrane,
- a faculative adhesive layer,
- a removeable protection layer and
- pergolid or one of its pharmaceutically acceptable salts as active agent.

16. Transdermal therapeutic system of Claim 15, **characterized by** known stabilizers, emulsifying agents, thickening agents and/or known membrane system additives.

17. Transdermal therapeutic system of Claim 15 or 16, **characterized by** a membrane on the basis of an inert polymer, in particular polypropylene, polyvinyl acetate or silicone.

## Revendications

1. Système thérapeutique transdermique contenant du pergolide ou de l'un de ses sels admissibles en pharmacie, ainsi que de la vitamine E naturelle, de la vitamine E synthétique et/ou un dérivé de vitamine E, servant d'agent accélérant la perméation.

2. Système thérapeutique transdermique conforme à la revendication 1, **caractérisé en ce que** la substance active est du pergolide à l'état de base libre, du mésylate de pergolide et/ou du chlorhydrate de pergolide.

3. Système thérapeutique transdermique conforme à la revendication 1 ou 2, **caractérisé en ce qu'**il contient au moins une autre substance active usuelle, à savoir au moins un autre agoniste de la dopamine.

4. Système thérapeutique transdermique conforme à la revendication 3, **caractérisé en ce qu'**il contient du pergolide ou de l'un de ses sels admissibles en pharmacie, en tant que substance active, en combinaison double ou triple avec d'autres agonistes de la dopamine.

5. Système thérapeutique transdermique conforme à l'une des revendications 3 et 4, **caractérisé en ce qu'**il contient du lévodopa, du carbidopa, de la sélégaline, de la tacrine, de la physostigmine, de la galanthamine ou de la 1-hydroxytacrine, et/ou de leurs dérivés chimiques, métabolites ou sels admissibles en pharmacie.

6. Système thérapeutique transdermique conforme à l'une des revendications précédentes, **caractérisé en ce qu'**il contient un autre agent accélérant la perméation, choisi parmi les alcools aliphatiques, cyclo-aliphatiques et/ou aromatique-aliphatiques, qui peuvent dans chaque cas être monofonctionnels ou polyfonctionnels et comporter jusqu'à 8 atomes de carbone, un mélange d'eau et d'alcool, les alcools gras saturés et/ou insaturés, pouvant dans chaque cas comporter de 8 à 18 atomes de carbone, les acides gras saturés et/ou insaturés, pouvant dans chaque cas comporter de 8 à 18 atomes de carbone, et/ou leurs esters.

7. Système thérapeutique transdermique conforme à la revendication 6, **caractérisé en ce qu'**il contient de l'éthanol, du 1,2-propanediol, du dexpanthénol et/ou un polyéthylèneglycol, en tant qu'agent accélérant la perméation.

8. Système thérapeutique transdermique conforme à Tune des revendications précédentes, **caractérisé en ce qu'**il contient un stabilisant connu, en particulier de la polyvinylpyrrolidone, du succinate d'α-tocophérol, du gallate de propyle, de la méthionine, de la cystéine et/ou du chlorhydrate de cystéine.

9. Système thérapeutique transdermique conforme à l'une des revendications précédentes, se présentant sous la forme d'un timbre comportant une couche imperméable de recouvrement et une couche protectrice amovible.

10. Système thérapeutique transdermique conforme à la revendication 9, **caractérisé en ce que** le timbre est un système à matrice ou un système à membrane.

11. Système thérapeutique transdermique conforme à la revendication 9 ou 10, **caractérisé en ce qu** comporte une couche de recouvrement à base de polyester, de polypropylène, de polyéthylène ou de polyuréthane, dans chaque cas éventuellement métallisée ou pigmentée.

12. Système thérapeutique transdermique conforme à la revendication 9, 10 ou 11, **caractérisé en ce qu'**il comporte une couche protectrice amovible à base de polyester, de polypropylène ou de papier revêtu de silicone et/ou de polyéthylène.

13. Système thérapeutique transdermique conforme à la revendication 9, 10, 11 ou 12, **caractérisé en ce qu'**il s'agit d'un système à matrice comportant
- une couche imperméable de recouvrement,
- une couche matrice auto-adhésive contenant les substances actives, ou une couche matrice contenant les substances actives et revêtue d'un adhésif,
- et une couche protectrice amovible,
- et **en ce qu'**il contient, en tant que substance active, du pergolide ou de l'un de ses sels admissibles en pharmacie.

14. Système thérapeutique transdermique conforme à la revendication 13, **caractérisé en ce que** la couche matrice est à base de polyacrylate, de silicone, de polyisobutylène, de caoutchouc butyl, de copolymère de styrène et de butadiène, ou de copolymère de styrène et d'isoprène.

15. Système thérapeutique transdermique conforme à la revendication 9, 10, 11 ou 12, **caractérisé en ce qu'**il s'agit d'un système à membrane comportant
- une couche imperméable de recouvrement,
- un réservoir contenant les substances actives, ou une couche réservoir contenant les substances actives,
- une membrane microporeuse ou semi-perméable,
- une couche facultative d'adhésif,
- et une couche protectrice amovible,
- et **en ce qu'**il contient, en tant que substance active, du pergolide ou de l'un de ses sels admissibles en pharmacie.

16. Système thérapeutique transdermique conforme à la revendication 15, **caractérisé en ce qu'**il contient des stabilisants, émulsifiants et/ou épaississants connus et/ou des adjuvants connus de systèmes à membrane.

17. Système thérapeutique transdermique conforme à la revendication 15 ou 16, **caractérisé en ce que** la membrane est à base de polymère inerte, en particulier de polypropylène, de poly(acétate de vinyle) ou de silicone.
